(19) Europäisches Patentamt
European Patent Office
Office européen des brevets

(11) Publication number: **0 166 026**
**A2**

(12) # EUROPEAN PATENT APPLICATION

(21) Application number: **84114239.1**

(22) Date of filing: **25.11.84**

(51) Int. Cl.$^4$: **C 07 D 277/34**

(30) Priority: **27.06.84 IT 2162184**

(43) Date of publication of application: **02.01.86**
**Bulletin 86/1**

(84) Designated Contracting States: **AT BE CH DE FR GB IT LI LU NL SE**

(71) Applicant: **CHEMI S.p.A., Via Gallarate, 184, I-20151 Milan (IT)**

(72) Inventor: **Benigni, Fulvio, Via Gallarate 184, I-20151 Milano (IT)**
Inventor: **Lissoni, Antonio, Via Gallarate 184, I-20151 Milano (IT)**
Inventor: **Sala, Bruno, Via Gallarate 184, I-20151 Milano (IT)**

(74) Representative: **Bianchetti, Giuseppe, Studio Consulenza Brevettuale Via Senato 24, I-20121 Milan (IT)**

(54) **Process for the preparation of N-thiocarboxyanhydrides of amino acids.**

(57) An improved process for the preparation of N-thiocarboxyanhydrides of amino acids, characterized by reacting a N-ethoxythiocarbonyl amino acid and phosphorus trihalide in the presence of methylterbutyl ether, is described.

EP 0 166 026 A2

"Process for the preparation of N-thiocarboxyanhydrides of amino acids"

The present invention relates to an improved process for the preparation of N-thiocarboxyanhydrides of amino acid, particularly N-thiocarboxyanhydride of L-aspartic acid.

This compound is an useful intermediate for the synthesis of Aspartame, a new sweetening agent, and generally for the synthesis of peptides, and it has been described in literature and patents.

The process for the preparation of N-thiocarboxyanhydrides of amino acids is disclosed in J. Org. Chem. 36, 49 (1971); in US patent No. 4,256,897 (Pfizer) and US patent No. 3,846,398 (Merck). Such a process is usually carried out by reacting a N-ethoxythiocarbonyl amino acid with a phosphorus trihalide ($PCl_3$ or $PBr_3$) in an inert organic solvent.

More particularly, the N-thiocarboxyanhydride of L-aspartic acid is obtained by reacting N-ethoxythiocarbonyl aspartic acid with $PBr_3$ or $PCl_3$, at temperatures ranging from 0 to 25°C, according to the following reaction scheme:

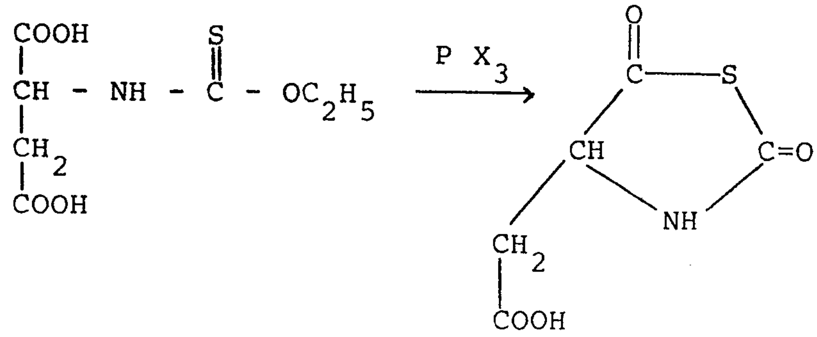

wherein X = Cl, Br.

US patent No. 4,256,897 describes such a synthesis, claiming the use of ethyl acetate as the solvent.

However, the process above described has a serious drawback, namely the production of a series of sulphur containing by-products which contaminate the obtained N-ethoxythiocarbonylanhydride, giving the compound a very unpleasant odour, which is often present also in the Aspartame obtained starting from said anhydride.

It has now surprisingly been found, and it is the object of the present invention, that a N-thiocarboxyanhydride of L-aspartic acid of high purity can be obtained in very high yields when carrying out the reaction between N-ethoxythiocarbonyl aspartic acid and phosphorus trihalide in the presence of methylterbutyl ether as the solvent.

This improved process gives an anhydride completely free from residual unpleasant odours, which compound allows to obtain Aspartame of very high quality.

The following example further illustrates the invention, without limiting it.

EXAMPLE

20 Kg of N-ethoxythiocarbonyl L-aspartic acid were dissolved in 80 liters of methylterbutyl ether. The reaction mixture was cooled to a temperature of 0 ÷ 5°C and 3.2 liters of $PBr_3$ were fastly added.

The temperature was allowed to raise spontaneou

sly and when it reached 25°C, the L-aspartic thiocarbonyl anhydride started to crystallize.

The reaction mixture was stirred for 30 minutes, then cooled again to 0 ÷ 5°C and left to stand for 15 minutes.

The obtained precipitate was filtered, washed with cold methylterbutyl ether and dried at 55°C, to give 14.5 kg of the compound. Yield: 91.6% of the theoretical.

($\alpha$ ($^{20°C}_{D}$ = -101.5 (C=1 in THF) ($\alpha$ reported in literature: -109).

CLAIMS for the Contracting States:

AT, BE, CH, DE, FR, GB, IT, LI, LU, NL, SE

1. A process for the preparation of N-thiocarboxyanhydrides of amino acids, starting from N-ethoxythiocarbonyl amino acids and phosphorus trihalides, characterized by carrying out the reaction in methylterbutyl ether.

2. A process according to claim 1, for the preparation of N-thiocarboxyanhydride of L-aspartic acid starting from N-ethoxythiocarbonyl L-aspartic acid and phosphorus trihalide, characterized by carrying out the reaction in methylterbutyl ether.

3. A process according to claims 1 and 2, wherein $PBr_3$ or $PCl_3$ are employed as the phosphorus trihalide.